# EUROPÄISCHE PATENTSCHRIFT

(11) **EP 1 180 043 B1**
(45) Veröffentlichungstag und Bekanntmachung des Hinweises auf die Patenterteilung: **16.07.2003**
(21) Anmeldenummer: 00945561.9
(22) Anmeldetag: 23.05.2000
(51) Int. Cl.: A61K 49/18

(54) **MEHRSTUFEN-VERFAHREN ZUR HERSTELLUNG VON GASGEFÜLLTEN MIKROKAPSELN**
MULTI-STAGE METHOD FOR PRODUCING GAS-FILLED MICROCAPSULES
PROCEDE A PLUSIEURS PHASES POUR LA PRODUCTION DE MICROCAPSULES REMPLIES DE GAZ

(30) Priorität: 27.05.1999 DE 19925311
(43) Veröffentlichungstag der Anmeldung: 20.02.2002
(73) Patentinhaber: SCHERING AKTIENGESELLSCHAFT, 13353 Berlin (DE)
(72) Erfinder: BUDDE, Uwe, D-16348 Wandlitz (DE); BRIEL, Andreas, D-10245 Berlin (DE); RÖSSLING, Georg, D-16548 Glienicke (DE); LOVIS, Kai, D-12169 Berlin (DE); SCHMIDT, Wolfgang, D-13595 Berlin (DE); MORITZ, Hans-Ulrich, D-21227 Bendestorf (DE); GOTTFRIED, Michael, D-20255 Hamburg (DE); INGWERSEN, Jan-Peter, D-13465 Berlin (DE)
(86) Internationale Anmeldenummer: DE0001678
(87) Internationale Veröffentlichungsnummer: WO00072888

(56) Entgegenhaltungen:
- WO-A-94/07539
- WO-A-95/07072
- DE-A- 4 004 430
- DE-A- 4 219 723
- DE-A- 4 219 724
- DE-A- 4 403 789
- DE-A- 19 648 663
- US-A- 5 501 863
- HARRIS J R ET AL: "The structure of gas-filled n-Butyl-2-cyanoacrylate (BCA) polymer particles." MICRON, Bd. 26, Nr. 2, 1995, Seiten 103-111, XP000952692
- SOMMERFELD P ET AL: "LONG-TERM STABILITY OF PBCA NANOPARTICLE SUSPENSIONS SUGGESTS CLINICAL USEFULNESS" INTERNATIONAL JOURNAL OF PHARMACEUTICS,NL,AMSTERDAM, Bd. 155, Nr. 2, 26. September 1997 (1997-09-26), Seiten 201-207, XP002066668 ISSN: 0378-5173
- VANDELLI M.A. ET AL: "Release behaviour of polyethyl- and polyisobutylcyanoacrylate nanoparticles as a function of surfactant used in the preparation procedure" BOLLETTINO CHIMICO FARMACEUTICO, 1993, VOL. 132, NO. 10, PAGE(S) 428-431, XP000952689 IT
- DOUGLAS S.J. ET AL: "Poly(butyl 2-cyanoacrylate) nanoparticles with differing surface charges" JOURNAL OF CONTROLLED RELEASE, 1986, VOL. 3, NO. 1, PAGE(S) 15-23, XP002150486
- SOMMERFELD P ET AL: "Long-term stability of PBCA nanoparticle suspensions." JOURNAL OF MICROENCAPSULATION, JAN-FEB 2000, VOL. 17, NO. 1, PAGE(S) 69-79, XP002150487
- DOUGLAS S J ET AL: "BIODISTRIBUTION OF POLY(BUTYL 2-CYANOACRYLATE) NANOPARTICLES IN RABBITS" INTERNATIONAL JOURNAL OF PHARMACEUTICS,AMSTERDAM,NL, Bd. 34, 1986, Seiten 145-152, XP000946109 ISSN: 0378-5173

## Beschreibung

Die Erfindung betrifft ein Mehrstufen-Verfahren zur Herstellung von gasgefüllten Mikrokapseln, bei dem die Verfahrensschritte Polymerisation der hüllgebenden Substanz und Mikrokapselaufbau getrennt erfolgt. Die mit dem erfindungsgemäßen Verfahren hergestellten Mikrokapseln besitzen eine Kern-Schale-Struktur und zeichnen sich durch eine definierte Größenverteilung aus. Aufgrund ihrer Eigenschaften können sie als kapillargängiges Kontrastmittel für Ultraschall eingesetzt werden.

Der Anmeldung liegen folgende Begriffsdefinitionen zugrunde:

Eine **Mikrokapsel** ist ein Teilchen im Größenbereich einiger µm bestehend aus einem gasförmigen Kern und einer festen Hülle mit variabler Dicke. Der Kern kann auch einen kleinen Anteil der Flüssigkeit, in der die Herstellung erfolgt, enthalten.

**Rühren** ist das Vermischen einer Flüssigkeit mit einer flüssigen, festen oder gasförmigen Substanz in der Weise, daß der Gasphasenanteil im Rührmedium < 1 % ist.

**Dispergieren** ist das Vermischen einer Flüssigkeit mit einer flüssigen, festen oder gasförmigen Substanz in der Weise, daß der Gasphasenanteil im Dispergiermedium > 1 %, vorzugsweise größer 10 % ist.

**Dispersion** ist ein kolloid- (Partikelgröße < 500 nm) oder grobdisperses (Partikelgröße > 500 nm) Mehrphasensystem

**Primärdispersion** ist eine kolloidale Dispersion aus Polymerpartikeln, hergestellt durch Polymerisation eines Monomers.

**Selbstbegasung** ist der Gaseintrag in eine Flüssigkeit durch dessen Bewegung oder durch das Herstellen eines dynamischen Strömungsunterdruckes.

**Fremdbegasung** ist der aktive Gaseintrag in eine Flüssigkeit.

**Flotation** ist die der Beschleunigngskraft (Erdbeschleunigng **g**, Radialbeschleunigung **a**) entgegen gerichtete Bewegung von Mikropartikeln aufgrund eines Dichteunterschiedes zwischen Mikropartikeln und Dispersionsmittel.

**Flotat** ist die aufgerahmte Schicht gasgefüllter Mikropartikel nach Flotation.

**Hydraulisch gefüllt** ist gleich vollständig ohne Gasüberstand gefüllt.

Bei der Echokardiographie (auch: Herzsonographie) können Rückschlüsse auf die Morphologie und Bewegungsabläufe der Herzklappen sowie die Richtung, Geschwindigkeit und Qualität der Blutströmung gezogen werden. Bei diesem Diagnoseverfahren wird mit Ultraschall gearbeitet, dessen Wechselwirkung farbcodiert dargestellt werden (Doppler-Verfahren). Wegen ihrer komplikationsarmen, einfachen Anwendung hat die Ultraschalldiagnostik in der Medizin weite Verbreitung gefunden.

Die Qualität der Ergebnisse wird durch den Einsatz von Kontrastmitteln deutlich verbessert.

Als Kontrastmittel werden in der medizinischen Ultraschalldiagnostik in der Regel Substanzen verwendet, die Gase enthalten oder freisetzen, da mit ihnen ein effizienterer Dichte- und damit Impedanzunterschied als zwischen Flüssigkeiten bzw. Feststoffen und Blut erzeugt werden kann.
Die Beobachtung cardialer Echoeffekte mit Lösungen, die fein verteilte Gasblasen enthalten, sind seit längerem literaturbekannt [1]. Da diese unstabilisierten Gasblasen nur eine sehr kurze Lebensdauer besitzen, sind auf diese Weise hergestellte Lösungen als Kontrastmittel zur medizinischen Ultraschalldiagnostik ungeeignet.

In dem US-Patent 4,276,885 wird ein Verfahren zur Herstellung von Gasbläschen beschrieben, die durch eine Gelatinemembran vor dem Zusammenfließen geschützt sind [2]. Diese Mikrobläschen werden bevorzugt durch eine Injektion des gewünschten Gases in eine gelierfähige Substanz (beispielsweise Gelatine) mittels einer Kapillare hergestellt. Eine Lagerung dieser Mikrobläschen ist nur bei tiefen Temperaturen möglich, wobei diese vor dem in-vivo Einsatz wieder auf Körpertemperatur zu bringen sind. Eine Hitzesterilisation ist prinzipiell ausgeschlossen, da dabei die Mikrobläschen ebenso zerstört werden wie bei einer Sterilfiltration.

In der Europäischen Patentschrift EP 0 052 575 B1 werden Ultraschallkontrastmittel beschrieben, welche auf physiologisch gut verträglichen Feststoffaggregaten basieren, die nach Applikation in den Blutstrom Gasblasen freisetzen [3]. Die freigesetzten Gasblasen sind nicht stabilisiert und überstehen keine Lungenpassage, so daß nach intravenöser Applikation nur eine Kontrastierung der rechten Herzhälfte möglich ist.

In den Patentschriften EP 0 122 624 und EP 0 123 235 werden Ultraschallkontrastmittel beschrieben, die aus Mikropartikeln und Gasbläschen bestehen [4, 5]. Im Gegensatz zu EP 0 052 575 B1 erfolgt eine Stabilisierung der Gasbläschen mittels einer grenzflächenaktiven Substanz. Eine Lungenpassage ist möglich, so daß diese Kontrastmittel eine Kontrastierung des gesamten Gefäßvolumens zulassen.
Beide Herstellverfahren sind allerdings sehr aufwendig.

Gemäß der Europäischen Patentschrift EP 0 324 938 B1 können gekapselte Mikrobläschen hergestellt werden, indem durch Ultraschall in einer Proteinlösung Mikrobläschen erzeugt werden, die nachfolgend dadurch stabilisiert werden, daß infolge eines lokalen Temperaturanstiegs das Protein teilweise denaturiert und die Gasbläschen einschließt [6].
Die vorgeschlagene Verwendung von Humanserumalbumin (HSA) birgt allerdings ein nicht unerhebliches allergenes Risiko.

In der Europäischen Patentschrift EP 0 398 935 B1 werden Mikropartikel als Ultraschallkontrastmittel beschrieben, deren Hüllsubstanz aus synthetischem, bioabbaubarem polymeren Material besteht. Als Hüllsubstanz kommen dabei eine ganze Reihe von Polymeren in Frage, die in einem mit Wasser nicht mischbaren Lösungsmittel oder Lösungsmittelgemisch gelöst werden und nach möglicher Zugabe weiterer Lösungsmittel in Wasser emulgiert werden. Als Lösungsmittel können gemäß [7] neben anderen Furan, Pentan und Aceton verwendet werden. In einer Verfahrensvariante wird das in einem der oben genannten Lösungsmitteln gelöste Monomer direkt in einer Gasblasen enthaltenden, wäßrigen Lösung polymerisiert.
Bei allen in den Ansprüchen genannten Verfahren ist die obligatorische Verwendung eines organischen Lösungsmittels von erheblichem Nachteil, da dieses im Verlauf des Herstellprozesses vollständig entfernt werden muß.

Mit den in der Europäischen Patentschrift EP 0 458 745 offenbarten Techniken lassen sich gasgefüllte Mikroballone in einem weiten Größenbereich herstellen [8]. Dazu wird zunächst eine Lösung des formgebenden Polymers in einem organischen Lösungsmittel in Wasser emulgiert und anschließend verdünnt, wodurch sich die fein verteilten Polymerlösungstropfen verfestigen. Das eingeschlossene Lösungsmittel muß in einem weiteren Schritt aufwendig entfernt werden. Vorteilhaft ist bei diesem Verfahren, daß man durch die Wahl des Tensids bzw. der Rührerdrehzahl eine direkte Einflußmöglichkeit auf die Größe der entstehenden Mikrokapseln besitzt. In diesem Fall sollen durch das Verfahren jedoch so unterschiedliche Applikationsformen wie die intravenöse Injektion, welche besonders kleine Partikel für eine Lungenpassage voraussetzt, sowie die orale Anwendung mit entsprechend größeren Partikeln abgedeckt werden. Eine lösungsmittelfreie Synthese von gasgefüllten Mikropartikeln ist auf diese Weise jedoch ebenfalls nicht möglich.

Ein Sprühtrocknungsverfahren zur Herstellung von echogenen Mikropartikeln, deren vornehmliches Charakteristikum konkave Oberflächensegmente sind, wird in der Europäischen Patentschrift EP 0 535 387 B1 offenbart [9]. Es wird u.a. die Synthese verschiedener Hüllpolymere unter Einsatz organischer Lösungsmittel beschrieben. Die echogenen Mikropartikel werden durch einen Sprühtrocknungsprozeß einer organischen Lösung des formgebenden Polymers gewonnen. Nachteilig bei diesem Verfahren ist ebenfalls die Verwendung organischer Lösungsmittel und das unter sterilen Bedingungen aufwendige Sprühtrocknungsverfahren.

Durch eine Verfahrensoptimierung, welche in der Europäischen Patentschrift EP 0 644 777 B1 beschrieben wird, konnte die Ultraschallaktivität der in der EP 0 327 490 beschriebenen Mikrokapseln deutlich verbessert werden [10]. Eine Zunahme der Ultraschallaktivität (bei gegebener Frequenz und kleiner Amplitude) wird erreicht, indem bei konstantem Partikeldurchmesser der Durchmesser des Luftkerns vergrößert wurde. Trotz der daraus resultierenden geringeren Wandstärke überdauern die Partikel dennoch eine Herz-Lungenpassage.
Das optimierte Verfahren ist dadurch gekennzeichnet, daß das Monomer in einer sauren, mit Gas gesättigten, wäßrigen Lösung dispergiert und polymerisiert wird und dabei direkt der Mikrokapselaufbau erfolgt. Auf diese Weise lassen sich Mikrokapseln herstellen, ohne daß man auf organische Lösungsmittel während des Herstellprozesses angewiesen ist.

Schwierigkeiten ergeben sich bei diesem Verfahren jedoch beim scale-up vom Labormaßstab auf den Produktionsmaßstab, da der Energieeintrag in das Reaktionsmedium in erheblichem Maß von der Umdrehungsgeschwindigkeit und dem Durchmesser des Rühr- bzw. Dispergierorgans abhängt. Demzufolge ist zu erwarten, daß die sensiblen Verhältnisse von Energie- und Lufteintrag lokal am Dispergierwerkzeug sowie des Schergefälles innerhalb des Reaktors nicht ohne weiteres maßstabsvergrößert werden können. Durch den hohen Lufteintrag des Dispergierwerkzeuges ist eine erhebliche Schaumbildung zu beobachten, so daß nur unzulängliche Aussagen getroffen werden können, inwiefern die Polymerisation des Monomers und die Hüllenbildung auf bestimmungsgemäße Weise erfolgt sind.

Der Erfindung liegt die Aufgabe zugrunde, ein Herstellverfahren für echogene Mikrokapseln zu finden, das keines der obengenannten Nachteile aufweist, d.h.
- die Herstellung der Mikrokapseln muß auch unter sterilen Bedingungen einfach und reproduzierbar sein,
- die Synthese des Polymers und die Mikrokapselherstellung muß ohne organische Lösungsmittel durchführbar sein,
- eine Maßstabsvergrößerung muß unter Erhalt der Prozeßkontrolle möglich und die Prozeßüberwachung leicht sein,
- die mit dem Verfahren herstellbaren Mikrokapseln sollen ein optimal angepaßtes Eigenschaftsprofil als Ultraschallkontrastmittel besitzen (definierte Größe bzw. Größenverteilung, qualitativ und quantitativ reproduzierbare Ultraschallkontraste),
- die Mikrokapseln sollten eine eine hohe Lagerstabilität auch unter kritischen klimatischen Bedingungen aufweisen.

Diese Aufgabe wird durch die vorliegende Erfindung gelöst.

Es wurde gefunden, daß nicht nur naszierende Primärlatexteilchen während des Polymerisationsprozesses Mikrokapseln bilden, sondern daß sich auch mit aus- bzw. vorpolymerisierten Polymerdispersionen durch geeignete Prozeßführung überraschenderweise eine Mikrokapselbildung hervorrufen läßt.
Durch diese Herstellmöglichkeit läßt sich der vergleichsweise komplizierte Gesamtherstellprozeß in kleinere Schritte zerlegen. Somit unterliegt der Gesamtherstellprozeß einer besseren Kontrolle.

Der Gegenstand der Erfindung betrifft daher ein mehrstufiges Verfahren zur Herstellung von gasgefüllten Mikrokapseln, bei dem in einem ersten Verfahrensschritt eine Polymerisation der hüllenbildenden Substanz und davon in einem räumlich und/oder zeitlich getrennten Verfahrensschritt die Bildung der Mikrokapseln durch einen Aufbauprozeß erfolgt. Die Teilprozesse Polymerisation und Mikrokapselbildung erfolgen somit getrennt.

Die Polymerisation des Monomers erfolgt dabei in wäßriger, oft saurer Lösung unter Rührbedingungen so, daß der Gasphasenanteil im Rührmedium < 1 % ist. Dies sind in der Regel moderate Bedingungen, in einem offenen Reaktor gekennzeichnet durch einen Energieeintrag von weniger als 5 W/dm³ und einer Reynoldszahl (Re=n·d²/ν) kleiner als 50000. Erfolgt die Polymerisation in einem beispielsweise hydraulisch gefüllten, geschlossenen System, ist eine bestimmungsgemäße Polymerisation auch bei deutlich anderen Betriebspunkten durchführbar.
In jedem Fall ist eine Trombenbildung wenn, nur gering erkennbar.
Als Zwischenprodukt dieses Verfahrensschrittes wird eine Primärdispersion aus kolloidalen Polymerpartikeln erhalten.

Der Mikrokapselaufbau aus dieser Polymerprimärdispersion erfolgt unter Dispergierbedingungen so, daß der Gasphasenanteil > 1 %, bevorzugt größer 10 % ist. Dies sind in der Regel Bedingungen, gekennzeichnet durch einen hohen Energieeintrag von mehr als 5 W/dm³ und einer Reynoldszahl (Re=n·d²/ν) größer als 50000. Eine Trombenbildung ist deutlich zu erkennen.
Es erfolgt in diesem Verfahrensschritt eine gerichtete strukturaufbauende Aggregation der kolloidalen Polymerpartikel.

Die signifikante Verfahrensverbesserung bei der Herstellung von Mikrokapseln liegt darin begründet, daß jeder einzelne Teilschritt unter den jeweils optimalen Prozeßbedingungen, wie zum Beispiel Temperatur, pH-Wert, Schereinwirkungen usw., durchgeführt werden kann.
So besteht die Möglichkeit, zunächst eine zur Bildung von Mikrokapseln optimal geeignete Primärdispersion des Hüllpolymers zu erzeugen, um dann nach dem Einstellen der für die Bildung der Mikrokapseln optimalen Bedingungen diese in einem weiteren Prozeßschritt herzustellen. Dieses kann vorteilhafterweise direkt im Anschluß an die Polymerisation erfolgen.

Als Monomere können Lactide, Alkylester der Acrylsäure, Alkylester der Methacrylsäure und bevorzugt Alkylester der Cyanacrylsäure eingesetzt werden.
Besonders bevorzugt sind Butyl-, Ethyl- und Isopropylcyanacrylsäure.

Das Rühr- bzw. Dispergiermedium kann ein oder mehrere der folgenden Tenside enthalten:
Alkylarylpoly(oxyethylen)sulfat Alkali-Salze, Dextrane, Poly(oxyethylene), Poly(oxypropylen)-poly(oxyethylen)-Blockpolymere, ethoxylierte Fettalkohole (Cetomacrogole), ethoxylierte Fettsäuren, Alkylphenolpoly(oxyethylene), Copolymere von Alkylphenolpoly(oxyethylen)en und Aldehyden, Partialfettsäurester des Sorbitans, Partialfettsäureester des Poly(oxyethylen)sorbitans, Fettsäureester des Poly(oxyethylen)s, Fettalkoholether des Poly(oxyethylen)s, Fettsäureester der Saccharose oder Macrogolglycerolester, Polyvinylalkohole, Poly(oxyethylen)-hydroxyfettsäureester, Macrogole mehrwertiger Alkohole, Partialfettsäureester.

Bevorzugt werden ein oder mehrere der folgenden Tenside eingesetzt:
ethoxylierte Nonylphenole, ethoxylierte Octylphenole, Copolymere von Aldehyden und Octylphenolpoly(oxyethylen), ethoxylierte Glycerin-Partialfettsäureester, etoxyliertes hydriertes Rizinusöl, Poly(oxyethylen)-hydroxystearat, Poly(oxypropylen)-poly(oxyethylen)- )-Blockpolymere mit einer Molmasse < 20000.

Besonders bevorzugte Tenside sind:
para-Octylphenol-poly-(oxyethylen) mit im Mittel 9-10 Ethoxygruppen (=Octoxynol 9,10), para-Nonylphenol-poly(oxyethylen) mit im Mittel 30/40 Ethoxygruppen (= z.B. Emulan®30,/Emulan®40), para-Nonylphenolpoly(oxyethylen)-sulfat-Na Salz mit im Mittel 28 Ethoxygruppen (= z.B. Disponil® AES), Poly(oxyethylen)glycerinmonostearat (z.B Tagat® S), Polyvinylalkohol mit Polymerisationsgrad von 600-700 und einem Hydrolysegrad 85 % - 90 % (= z.B. Mowiol® 4-88), Poly(oxyethylen)-660-Hydroxystearinsäureester (= z.B. Solutol® HS 15), Copolymer von Formaldehyd und para-Octylphenolpoly(oxyethylen) (= z.B. Triton® WR 1339), Polyoxypropylen-polyoxyethylen-Blockpolymere mit einer Molmasse von ca. 12000 und einem Polyoxyethylen-Anteil von ca. 70% (= z.B. Lutrol® F127), ethoxylierter Cetylstearylalkohol (= z.B. Cremophor® A25), ethoxyliertes Rizinusöl (= z.B. Cremophor® EL).

Generell kann die Herstellung der gasgefüllten Mikrokapsln im koninuierlichen, halb kontinierlichen oder im Satz Betrieb erfolgen. Für die Polymerisation wird eine Kombination eines oder mehrerer, gleicher oder verschiedener Reaktoren vom Typ eines Rührkessels, eines Strömungsrohres oder eine Schlaufenreaktors, mit geeigneter Vorkehrung zur Durchmischung eingesetzt.

Für die Erzeugung der gasgefüllten Mikrokapseln besitzt der eingesetzte Reaktor eine geeignete Dispergiereinehit und die Möglichkeit ein entsprechenden Gaszutritt in das Reaktionsmedium zu erlauben.

In der bevorzugten Verfahrensvariante wird im Verfahrensschritt der Polymerisation ein Monomer aus der Gruppe der Cyanacrylsäurealkylester in eine saure wäßrige Lösung getropft. Die Zugabe erfolgt unter so moderaten Rührbedingungen, daß keine Selbstbegasung erfolgt.

Als diskontinuierlicher Reaktor eignet sich besonders ein Rührkessel mit einem Verhältnis von Durchmesser zu Höhe in einem Bereich von 0,3 bis 2,5, der mit einem Temperiermantel ausgestattet ist.

Die Durchmischung wird bevorzugt mit einem Rührorgan realisiert, welches ein Verhältnis von Rührer- zu Reaktordurchmesser in einem Bereich von 0,2 bis 0,7 besitzt.
Als Rührorgane kommen prinzipiell alle üblichen Rührer in Betracht, insbesondere aber solche, wie sie für die Durchmischung niedrigviskoser, wasserähnlicher Medien (< 10 mPas) eingesetzt werden. Dazu zählen beispielsweise Propeller-, Blatt-, Schrägblatt-, MIG®-, Scheibenrührer usw. Die Einbaulage kann z.B. vertikal in Richtung der Normalen der Flüssigkeitsoberfläche des Reaktionsmediums, in schräger Form gegen die Normale oder seitlich durch die Behälterwand erfolgen. Letztere Möglichkeit ergibt sich bei einem vollständig gasfrei gefüllten, nach außen zur Atmosphäre gekapselten Behälter.
Ferner ist der Einsatz von Strömungsbrechern möglich. Hierdurch wird gewährleistet, daß die Neigung zur Selbstbegasung in einem offenen System bei der Herstellung der Primärdispersion besonders gering ist.

Eine Entgasung der Reaktionsmedien kann, muß aber nicht erfolgen. Üblicherweise besitzen die Reaktionsmedien den temperaturabhängigen Gasgehalt des Gases (der Gase) der umgebenden Atmosphäre.
Insgesamt sollte die Herstellung so erfolgen, daß keine optisch erkennbare Zunahme des Volumens des Reaktionsmediums durch Gaseintrag erfolgt (Φ_{G}<<1 %).

Ferner sollte der Dosierungsort in Verbindung mit den anderen zur Durchmischung beitragenden Einbauten, der Rührer und die Rührerdrehzahl so gewählt werden, daß die Mischzeit im Vergleich zur Reaktionsdauer des Polymerisationsprozesses sehr klein ist, um eine möglichst schnelle Mikrovermischung des Monomeren in der sauren wäßrigen Lösung zu gewährleisten.

Durch die vergleichsweise überschaubare Hydrodynamik eines diskontinuierlichen Rührkessels gibt es bei einer Maßstabsvergrößerung vom Laboratoriums- auf den Technikums- bzw. den Produktionsmaßstab keine nennenswerten Schwierigkeiten, was als Vorteil für die kommerzielle Anwendung dieses Verfahrens gewertet werden muß.

Bei ordnungsgemäßer Durchführung ist keine Schaumbildung zu beobachten. Während der Polymerisation erfolgt kein oder ein nur sehr geringer Gaseintrag, und Kavitationseffekte sind aufgrund der moderaten Rührbedinungen ausgeschlossen. So ist es sehr leicht möglich, durch Einsatz geeigneter on-line Prozeßsonden (z.B. IR-, NIR- oder Raman-Sonden für den Umsatz), welche bei stark schäumenden Reaktionsmedien oft unbrauchbar sind, die Reaktionsführung und Prozeßkontrolle sicher zu gestalten.
Ferner ist es möglich, nach dem Reaktionsende die Polymerdispersion zu beproben und konventionell eine off-line Analytik durchzuführen. So kann dann z.B. die mittlere Teilchengröße und -verteilung bestimmt werden.

Eine weitere, ebenfalls erfolgreich durchgeführte Technik zur Einstellung von gewünschten Partikelgrößenverteilungen stellt der Zulauf von Monomeren während einer halbkontinuierlichen Polymerisation dar, damit das Wachstum einer in der Anfangsphase der Polymerisation generierten Teilchenpopulation gezielt beeinflußt wird.

Die Polymerisation wird bei Temperaturen von -10 °C bis 60 °C, bevorzugt in einem Bereich von 0 °C bis 50 °C und besonders bevorzugt zwischen 10 °C und 35 °C, durchgeführt.

Die Einstellung der Reaktionsgeschwindigkeit der Polymerisation der Cyanacrylsäureester und der daraus resultierenden mittleren Teilchengröße erfolgt u.a neben der Temperatur über den pH-Wert, der sich säure- und konzentrationsabhängig in einem Bereich von 1,0 bis 4,5, beispielsweise durch Säuren, wie Salzsäure, Phosphorsäure und/oder Schwefelsäure, einstellen läßt. Weitere Einflußgrößen auf die Reaktionsgeschwindigkeit sind die Art und Konzentration des Tensides und die Art und Konzentration von Zusatzstoffen.

Das Monomer wird in einer Konzentration von 0,1 bis 60 %, bevorzugt von 0,1 bis 10 % zur wäßrigen, meist sauren, Lösung zugesetzt.

Bei einer Durchführung nach den obengenannten Bedingungen liegt die Polymerisationszeit zwischen 2 Minuten und 2 Stunden und kann u.a. reaktionskalorimetrisch verfolgt werden. Dieser weite Bereich der Reaktionszeit ist eine Folge der flexiblen Variationsmöglichkeiten bei der Wahl der Prozeßparameter, mit denen die Partikelgröße sowie die Partikelgrößenverteilung der entstehenden Polymerlatexteilchen gesteuert werden kann. Dieses sind die zentralen Einflußgrößen bei der anschließenden Bildung der gasgefüllten Mikrokapseln, die somit durch die Wahl geeigneter Polymerisationsparameter positiv beeinflußt werden können.

Der Durchmesser der nach dieser Rezeptur hergestellten Polymerlatexteilchen für die Einkapselung von Gas liegt im Bereich von 10 nm bis 500 nm, bevorzugt im Bereich von 30 nm bis 150 nm, besonders günstig im Bereich von 60 nm bis 120 nm. Die so hergestellten Polymerteilchen besitzen eine steuerbare Größenverteilung mit einer Polydispersität bis hinunter zu einem Bereich von 1,4 bis 1,0 (d_{w}/dₙ).

Sterilitätsprobleme gibt es bei diesem einfachen Reaktionsaufbau nicht. Für die aseptische Fertigung von Mikrokapseln ist es möglich, diese Polymerdispersion einer Sterilfiltration zu unterziehen, so daß der aseptische Fertigungsprozeß einfach zu realisieren ist.

Im Anschluß an die Polymerisation kann als weiterer Vorteil dieses mehrstufigen Verfahrens ein eventuell bei der Polymerisation entstehender Grobanteil abgetrennt werden (z.B. durch Filtration), so daß dieser nicht mehr störend auf den Bildungsprozeß der Mikrokapseln wirkt.

Neben anderen Prozeßschritten, wie die schon genannte Filtration, ist auch eine Dialyse möglich. Damit läßt sich der Tensidgehalt der Primärdispersion wieder absenken. Das Tensid kann dann für den nächsten Schritt, dem Aufbauprozeß von auspolymerisierten Latexteilchen zu Mikrokapseln, ganz oder teilweise durch ein anderes ersetzt werden. Außerdem können weitere Hilfsstoffe zugegeben werden.

Die Bildung der gasgefüllten Mikrokapseln erfolgt in einem weiteren Schritt durch strukturaufbauende Aggregation der kolloidalen Polymerteilchen. Dieser Verfahrensschritt erfolgt räumlich und/oder zeitlich getrennt von der Herstellung der Primärdispersion.
Dazu muß die Primärdispersion mit einem Dispergierwerkzeug so agitiert werden, daß der Phasenanteil des Gases Φ_{G} im Reaktionsgemsisch auf Werte deutlich über 1 %, im allgemeinen auf mehr als 10 % ansteigt. Der Gasphasenanteil im Medium beträgt oft sogar mehr als 50 %. Damit ist eine entsprechend starke Zunahme des Volumens der Reaktionsmischung verbunden. Es erfolgt eine intensive Schaumbildung, die über eine Transmissionsmessung durch einen Trübungssensor quantifizierbar ist.
Für die Herstellung gasgefüllter Mikrokapseln genügt bei hochtourigem Dispergieren der Gasraum über der entstehenden Trombe.

Der Mikrokapselaufbau wird bei Temperaturen von -10 °C bis 60 °C, bevorzugt in einem Bereich von 0 °C bis 50 °C und besonders bevorzugt zwischen 10 °C und 35 °C, durchgeführt.

Die Größe und die Größenverteilung dieser Mikrokapseln wird durch verschiedene Prozeßparameter, etwa das Schergefälle oder die Rührdauer bestimmt. Der Durchmesser der gasgefüllten Mikrokapseln liegt in einem Bereich von 0,2-50 µm, bei Parenteralia bevorzugt zwischen 0,5 und 10 µm und besonders bevorzugt zwischen 0,5 und 3 µm.

Als Dispergierwerkzeuge bei der Herstellung gasgefüllter Mikrokapseln eignen sich insbesondere Rotor-Stator-Mischer, die ein hohes Schergefälle erzeugen können. Zusätzlich gewährleisten sie in dem für die Herstellung der Mikrokapseln nötigen Zeitrahmen gleichzeitig einen hohen Gaseintrag.
Die Abmessungen und Betriebsgrößen des (der) Dispergierwerkzeugs(e) bestimmen wesentlich die Teilchengrößenverteilungen der Mikrokapseln, ferner richtet sich ihre Dimensionierung nach Größe und Kühlkapazität der Anlage.

Eine vorteilhafte Möglichkeit des erfindungsgemäßen mehrstufigen Verfahrens besteht darin, daß es nicht notwendig ist einen Ansatz vollständig durchzuprozessieren.
Das heißt, es besteht die Option mehrere verschiedene Primärdispersionen, die auch jeweils verschiedene Polymere beinhalten können, zusammenzuführen, um daraus gasgefüllte Mikrokapseln aufzubauen.
Ferner kann eine Primärdispersion auch in Portionen aufgeteilt werden, die dann jeweils für sich weiter zu gasgefüllten Mikrokapseln aufgebaut werden. Außerdem können für die nachfolgenden Verfahrensschritte notwendige bzw. optimal geeignete Hilfsstoffe zugesetzt werden.
Nach Beendigung der Bildung der Mikrokapseln stehen alle Möglichkeiten offen weiterzuprozessieren: z.B. die Abtrennung von gasgefüllter Mikrokapseln aufgrund des Dichteunterschieds zum flüssigen Medium. Bei hinreichend druckstabilen Mikrokapseln kann eine Zentrifugation erfolgen usw..

Das Eigenschaftsprofil kann besonders gut durch die Gliederung des Herstellprozesses in Teilschritte gesteuert werden.

Bei einer Maßstabsvergrößerung ergibt sich ebenfalls eine Verbesserung gegenüber dem Stand der Technik. Da die bei einem einstufigen Verfahren gekoppelten Prozesse von Durchmischung, Polymerisation und dem Aufbau von Mikrokapseln parallel ablaufen, müssen diese drei Prozesse simultan maßstabsvergrößert werden. Bei der Zerlegung des Gesamtprozesses in Einzelprozesse wird vorteilhaft ausgenutzt, daß eine Fokussierung auf die für den jeweiligen Prozeß wichtigen Größen wesentlich einfacher ist, weil auf Teilprozesse mit weniger charakteristischen Größen heruntergebrochen wird.

Für eine Maßstabsvergrößerung gilt nämlich immer, daß es unmöglich ist, alle, den Prozeß beschreibenden Größenverhältnisse wie Wärmeaustauschfläche zum Reaktorvolumen (A/V), oder Kenngrößen wie Durchmesser Rührer zu Durchmesser Reaktor (d_{Rührer}/d_{Reaktor}), Gasphasengehalt (Φ_{G}), Reynoldszahl (Re), Nußelt-Zahl (Nu), Newton-Zahl (Ne), Prandtl-Zahl (Pr), Reaktorhöhe/-Reaktordurchmesser (h/d) u.v.m gleichzuhalten.
Es tauchen hier auch Kennzahlen auf, die nur Stoffwerte beinhalten, etwa die Prandtl-Zahl, und die vom Scale-up eigentlich nicht betroffen sein sollten. Aber auch diese Stoffwerte, wobei es sich z.B. um die Wärmekapazität, Dichte, Viskosität oder die mittlere Wärmeleitzahl des Reaktionsmediums handelt, sind eine Funktion des relativen Gasphasengehaltes Φ_{G}, so daß alle diese Kennzahlen zusätzlich vom relativen Gasphasengehalt abhängen.

Wichtige Größen für die Herstellung der Primärdispersion und die Aufbaureaktion der Mikrokapseln sind in diesem Zusammenhang neben den fundamentalen Größen wie Temperatur, Rezeptur, etc. der Gasphasenanteil, der spezifische Energieeintrag und die Reynoldszahl. Letztere nehmen natürlich für beide Verfahrensschritte verschiedene Werte an. Für den Prozeßschritt des Mikrokapselaufbaus sind wegen des hohen spezifischen Energieeintrags dann insbesondere noch die thermische Kontrolle und die damit verbundenen (Kenn)Größen wie Nu(Φ_{G}), A/V, Wärme-übergangskoeffizient (α), Pr(Φ_{G}) usw. wichtig. Diese Überlegungen gelten auch für die im folgenden genannten möglichen Prozeßvarianten.

Eine konkrete Verfahrensvariante besteht darin, die Herstellung der Primärdispersion in einem kontinuierlichen Reaktor durchzuführen, wobei sich dazu Rohrreaktoren mit ihrem engen definierten Verweilzeitverhalten besser eignen als Rührkesselreaktoren. Durch die geeignete Wahl der Polymerisationsparameter, der Reaktorgeometrie und der mittleren Verweilzeit kann bei einem Rohrreaktor auf einfache Weise sichergestellt werden, daß die Polymerisation am Ende des Rohrreaktors vollständig abgelaufen ist. Ebenso wie beim batch Reaktor besteht die Möglichkeit einer on-line Analytik.

Ferner kann am Ende des Rohrreaktors ein mehrstufiges Rotor-Stator-System für die Aufbaureaktion von Mikrokapseln eingesetzt werden, so daß der gesamte Prozeß in einer einzigen Apparatur durchgeführt wird, und die beiden Prozeßschritte, die Herstellung einer Polymerdispersion und die Aufbaureaktion von Mikrokapseln, dennoch voneinander entkoppelt werden.

Eine weitere Prozeßvariante sieht die Verwendung eines Schlaufenreaktors vor, der aus einem kontinuierlichen oder gegebenenfalls aus einem diskontinuierlichen Rührkessel mit einer Außenschlaufe besteht, in der eine einoder mehrstufige inline-Dispergiereinheit bzw. ein ein- oder mehrstufiges Rotor-Stator-System untergebracht ist, welches zusätzlich die Förderleistung für die Außenschlaufe erbringen kann.
In diesem Fall erfolgt die Herstellung der Primärdispersion entweder im Rührkesselbereich unter den moderaten Rührbedingungen sowie bei geschlossener Schlaufe oder im gesamten Schlaufenreaktor bei geöffneter Schlaufe, und zwar unter Umlaufbedingungen, die durch entsprechend eingestellte Drehzahlbereiche keine Selbstbegasung erlauben.

Das Verfahren mit geöffneter Schlaufe bietet dabei den Vorteil, die Mikrovermischung des Monomeren in die wäßrige Lösung hinein durch gezielte Dosierung des Monomeren in den Zulaufbereich der Rotor-Stator-Einheit besonders vorteilhaft zu gestalten.
Nach Reaktionsende wird die Schlaufe entweder geöffnet, um dann durch die in der Schlaufe integrierte Rotor-Stator-Einheit die Aufbaureaktion von Mikrokapseln zu ermöglichen, oder es wird bei von Anfang an geöffneter Schlaufe der Drehzahlbereich der Rotor-Stator-Einheit entsprechend erhöht. Während dieses Verfahrensschrittes kann eine Selbstbegasung im Rührkesselbereich über eine Trombe oder auch eine Fremdbegasung in Form einer gezielten Dosierung im Zulaufbereich der Rotor-Stator-Einheit vorgenommen werden. Letztgenanntes Verfahren bietet den Vorteil eines genau kontrollierbaren Zulaufs.

Im Hinblick auf eine möglichst einfache Maßstabsvergrößerung des Prozesses zur Mikrokapselherstellung sind alle genannten Verfahren, die eine Rotor-Stator-Einheit im Umlaufrohr eines Schlaufenreaktors oder im Rohrteil eines kontinuierlichen Strömungsrohres haben, den reinen Rührkesselverfahren vorzuziehen. Ein Grund dafür ist, daß bei einer Vergrößerung des Rührkesselvolumens oder des zu produzierenden Volumens das in-line-Dispergiersystem nicht notwendigerweise entsprechend vergrößert werden muß, sondern lediglich die Betriebszeit angepaßt wird. Ein weiterer Grund ist in der Tatsache zu sehen, daß sich die Dispergierwirkung einer Rotor-Stator-Einheit in einem durchströmten Rohr allgemein leichter quantifizieren läßt als in einem Rührkesselreaktor, wodurch die Maßstabsvergrößerung sich ebenfalls deutlich vorteilhafter darstellt.

Nach Beendigung beider Verfahrensschritte kann der Reaktionsansatz weiter aufgearbeitet werden.
Empfehlenswert ist die Abtrennung der gasgefüllten Mikrokapseln vom Reaktionsmedium.
Dies kann in einfacher Weise unter Ausnutzung des Dichteunterschiedes durch Flotation oder Zentrifugation erfolgen. Die gasgefüllten Mikrokapseln bilden in beiden Fällen ein Flotat, das sich leicht vom Reaktionsmedium abtrennen läßt. Das gewonnene Flotat kann dann mit einem physiologisch verträglichen Trägermedium, im einfachsten Fall Wasser oder physiologische Kochsalzlösung, aufgenommen werden.
Die Suspension kann direkt appliziert werden. Gegebenenfalls ist eine Verdünnung empfehlenswert.
Der Separationsvorgang kann auch ein- oder mehrfach wiederholt werden. Durch gezielte Einstellung der Flotationsbedingungen lassen sich Fraktionen mit definierten Eigenschaften gewinnen.

Die Suspensionen sind über einen sehr langen Zeitraum stabil und die Mikrokapseln aggregieren nicht.
Die Haltbarkeit kann dennoch durch eine anschließende Gefriertrocknung gegebenenfalls nach Zusatz von Polyvinylpyrrolidon, Polyvinylalkohol, Gelatine, Humanserumalbumin oder einem anderen, dem Fachmann geläufigen, Kryoprotektors verbessert werden.

Die Erfindung wird durch folgende Beispiele erläutert:

### Beispiel 1

In einem 1 I Glasreaktor mit dem Verhältnis Durchmesser zu Höhe von 0,5 werden 800 ml Wasser durch Zugabe von 0,1n Salzsäure auf einen pH-Wert von 2,5 und eine Reaktortemperatur von 290,5 K eingestellt. Unter mäßigem, zur Vermeidung von Lufteintrag, Rühren mit einem Propellerrührer werden 8,0 g Octoxynol zugegeben und solange gerührt, bis das Octoxynol vollständig aufgelöst ist. Anschließend werden unter gleichen Rührbedingungen über einen Zeitraum von 5 Minuten 11,20 g Cyanacrylsäurebutylester zugetropft und die Lösung für weitere 30 Minuten gerührt. Nachdem die Polymerisation beendet ist, wird die Polymerdispersion zur Abtrennung größerer Polymerteilchen filtriert. Die filtrierte Dispersion wird 60 Minuten mit einem Rotor-Stator-Mischer bei hohen Schergefällen gemischt. Durch die intensive Mischung erfolgt eine Selbstbegasung des Prozeßmediums mit der Folge einer starken Schaumbildung. Es bildet sich nach Reaktionsende eine aufrahmende Schicht gasgefüllter Mikrokapseln aus.
Das Flotat wird vom Reaktionsmedium abgetrennt und mit 600 ml Wasser für Injektionszwecke aufgenommen. Anschließend werden 60 g Polyvinylpyrrolidon im Ansatz gelöst, die Suspension á 5 g konfektioniert und gefriergetrocknet.

### Beispiel 2

In einem 2 I Glasreaktor mit einem Verhältnis Durchmesser zu Höhe von ca. 0,5 und einer Außenschlaufe mit einer einstufigen Rotor-Stator-Mischeinheit wird 1 I einer Lösung von 1 % Octoxynol bei einem pH-Wert von 2,5 vorgelegt und anschließend über 5 Minuten 14 g Cyanacrylsäurebutylester zugetropft und die Lösung für 30 Minuten gerührt, ohne Luft in die Reaktionsmischung einzutragen.
Anschließend wird die Außenschlaufe für 60 min im Kreislauf zugeschaltet. Der Rührer im Glasreaktor wird so eingestellt, daß eine Selbstbegasung der Reaktionsmischung erfolgt. Es bildet sich nach dem Versuchsende eine aufrahmende Schicht.
Das Flotat wird vom Reaktionsmedium abgetrennt und mit 1,5 I Wasser für Injektionszwecke aufgenommen. Anschließend werden 150 g Polyvinylpyrrolidon im Ansatz gelöst, die Suspension á 10 g konfektioniert und gefriergetrocknet.

### Beispiel 3:

In einem 50 I Stahlreaktor mit einem Verhältnis Durchmesser zu Höhe von ca. 0,5 und einer Außenschlaufe mit einer dreistufigen Rotor-Stator-Mischeinheit werden 30 I einer Lösung von 1 % Octoxynol bei einem pH-Wert von 2,5 vorgelegt. Es werden 430 g Cyanacrylsäurebutylester in die Außenschlaufe direkt vor die Rotor-Stator-Mischeinheit zugegeben. Der Rotor-Stator wird dabei so betrieben, daß keine Selbstbegasung des Mediums erfolgt. Die Lösung wird für 30 Minuten in der Außenschlaufe umgepumpt.
Anschließend erfolgt eine Fremdbegasung in der Außenschlaufe mit Luft direkt vor der hochtourig laufenden dreistufigen Rotor-Stator-Mischeinheit. Die Lösung wird dabei für weitere 60 Minuten in der Außenschlaufe umgepumpt.
Es bildet sich nach Versuchsende eine aufrahmende Schicht.
Das Flotat wird vom Reaktionsmedium abgetrennt und mit 35 I Wasser für Injektionszwecke aufgenommen. Anschließend werden 3,5 kg Polyvinylpyrrolidon im Ansatz gelöst, die Suspension ä 7,5 g konfektioniert und gefriergetrocknet.

### Beispiel 4

In einem 1 I Glasreaktor mit dem Verhältnis Durchmesser zu Höhe von 0,5 werden 800 ml Wasser durch Zugabe von 0,1n Salzsäure auf einen pH-Wert von 1,50 und eine Reaktortemperatur von 288 K eingestellt. Unter mäßigem, zur Vermeidung von Lufteintrag, Rühren mit einem Propellerrührer werden 8,0 g eines Alkylarylethersulfats (Disponil AES 72) zugegeben und solange gerührt, bis das Tensid vollständig aufgelöst ist. Anschließend werden unter gleichen Rührbedingungen über einem Zeitraum von 5 Minuten 11,20 g Cyanacrylsäurebutylester zugetropft und die Lösung für 30 Minuten gerührt. Nachdem die Polymerisation beendet ist, wird die Polymerdispersion zur Abtrennung größerer Polymerteilchen filtriert.
Die filtrierte Dispersion wird 60 Minuten mit einem Ultraturrax bei hohen Schergefällen (etwa 14000 s⁻¹) behandelt. Durch die intensive Mischung erfolgt automatisch ein Eintrag von Luft in das Prozeßmedium mit der Folge einer starken Schaumbildung. Es bildet sich nach Reaktionsende eine aufrahmende Schicht gasgfüllter Mikrokapseln aus.
Das Flotat wird vom Reaktionsmedium abgetrennt und in 600 ml Wasser für Injektionszwecke aufgenommen. Anschließend werden 60 g Polyvinylpyrrolidon im Ansatz gelöst, die Suspension in Mengen à 5 g konfektioniert und gefriergetrocknet.

### Beispiel 5

In einem 1 I Glasreaktor mit dem Verhältnis Durchmesser zu Höhe von 0,5 werden 1000 ml Wasser durch Zugabe von 0,1n Salzsäure auf einen pH-Wert von 2,50 und eine Reaktortemperatur von 288 K eingestellt. Unter mäßigem, zur Vermeidung von Lufteintrag, Rühren mit einem Propellerrührer werden 10,0 g eines Polyvinylalkohols (Mowiol 4-88) zugegeben und solange gerührt, bis das Tensid vollständig aufgelöst ist. Anschließend werden unter gleichen Rührbedingungen über einem Zeitraum von 5 Minuten 14,0 g Cyanacrylsäurebutylester zugetropft und die Lösung für 45 Minuten gerührt. Nachdem die Polymerisation beendet ist, wird die Polymerdispersion zur Abtrennung größerer Polymerteilchen filtriert.
Die filtrierte Dispersion wird 60 Minuten mit einem Ultraturrax bei hohen Schergefällen (etwa 14000 s⁻¹) behandelt. Durch die intensive Mischung erfolgt automatisch ein Eintrag von Luft in das Prozeßmedium mit der Folge einer starken Schaumbildung. Es bildet sich nach Reaktionsende eine aufrahmende Schicht gasgfüllter Mikrokapseln aus.
Das Flotat wird vom Reaktionsmedium abgetrennt und in 1500 ml Wasser für Injektionszwecke aufgenommen. Anschließend werden 150 g Polyvinylpyrrolidon im Ansatz gelöst, die Suspension in Mengen ä 5 g konfektioniert und gefriergetrocknet.
¹ J. ROELANDT
   *Contrast echocardiography (review)*
   Ultrasound Med. Biol. **8**., S. 471-492, 1982
² US-Patent 4,276,885, 04.05.1979
   E.G. TICKNER ET. AL.
   *Ultrasonic Image Enhancement*
   Rasor Associates, Inc.
³ Europäisches Patent EP 0 052 575, 17.11.1980
   J. S. RASOR, E. G. TICKNER
   *Composition generating microbubbles*
   Schering AG
⁴ Europäisches Patent EP 0 122 624, 15.04.1983
   J. HILLMANN ET AL.
   *Mikrokapsel und Gasbläschen enthaltendes Ultraschallkontrastmittel*
   Schering AG
⁵ Europäisches Patent EP 0 123 235, 15.04.1983
   J. HILLMANN ET AL.
   *Mikrokapsel und Gasbläschen enthaltendes Ultraschallkontrastmittel*
   Schering AG
⁶ Europäische Patentschrift EP 0 324 938 B1, 29.12.1987
   K. J. WIDDER, P. J WESTKAEMPER
   *Concentrated stabilized microbubble-type ultrasonic imaging agent and method of production*
   Molecular Biosystems Inc.
⁷ Europäische Patentschrift EP 0 398 935 B1, 05.02.1988
   M. STEIN ET AL.
   *Ultraschallkontrastmittel, Verfahren zu deren Herstellung und deren Verwendung als*
   *Diagnostika und Therapeutika*
   Schering AG
⁸ Europäische Patentschrift EP 0 458 749, 18.05.1990
   D. BICHON ET AL.
   *Mit Gas oder Luft gefüllte polymere Mikrokapseln, verwendbar in Form von Suspension bei flüssigen Trägern für Ultraschall-Echographie*
   Bracco Int
⁹ Europäische Patentschrift EP 0 535 387 B1, 03.09.1990
   V. KRONE ET AL.
   *Echogene Partikel, Verfahren zu deren Herstellung und deren Verwendung*
   Hoechst AG
¹⁰ Europäisches Patent EP 0 644 777 B1, 13.06.1992
   M. STEIN ET AL.
   *Microparticles, method of producing them and their use for diagnostic purposes*
   Schering AG

## Patentansprüche

1. Mehrstufen-Verfahren zur Herstellung von gasgefüllten Mikrokapseln, **dadurch gekennzeichnet, dass** folgende Verfahrensschritte räumliche und/oder zeitlich getrennt erfolgen
a) Polymerisation der hüllenbildenden Substanz unter Bildung einer Primärdispersion aus kolloidalen Polymerpartikeln
b) Strukturaufbauende Aggregation der kolloidalen Polymerpartikel zu gasgefüllten Mikrokapseln.

2. Verfahren nach Anspruch 1, **dadurch gekennzeichnet, dass** die Polymerisation des Momomers in wässriger Lösung unter Rührbedingungen so erfolgt, dass der Gasphasenanteil im Rührmedium < 1 % ist.

3. Verfahren nach Ansprüchen 1 oder 2, **dadurch gekennzeichnet, dass** der Mikrokapselaufbau aus einer Polymerprimärdispersion unter Dispergierbedingungen so erfolgt, dass der Gasphasenanteil im Dispergiermedium > 1 %, bevorzugt großen als 10 % ist.

4. Verfahren nach einem der Ansprüche 1 bis 3, **dadurch gekennzeichnet, dass** die Polymerisation des Monomers in einem dis-, halboder kontinuierlichen Rührkessel mit einem Verhältnis Durchmesser zu Höhe von 0,3 bis 2,5 durchgeführt wird.

5. Verfahren nach einem der Ansprüche 1 bis 4, **dadurch gekennzeichnet, dass** die Polymerisation des Monomers in einem dis-, halboder kontinuierlichen Rührkessel in einem Verhältnis von Durchmesser zu Höhe von 0,3 bis 2,5 mit Außenschlaufe (Schlaufenreaktor) durchgeführt wird, in der eine ein- oder mehrstufige Dispergiereinheit angeordnet ist, welche zu Beginn der Reaktion oder später zugeschaltet wird.

6. Verfahren nach Ansprüchen 1 bis 5, **dadurch gekennzeichnet, dass** die Polymerisation des Monomers in einem vertikal, schräg oder seitlich angeordneten Rührorgan durchgeführt wird, dessen Durchmesser im Verhältnis zum Reaktordurchmesser in einem Bereich von 0,2 bis 0,7 liegt.

7. Verfahren nach Ansprüchen 1 bis 3, **dadurch gekennzeichnet, dass** die Polymerisation des Monomers in einem kontinuierlich betriebenen Strömungsrohrreaktor durchgeführt wird.

8. Verfahren nach Ansprüchen 1 bis 7, **dadurch gekennzeichnet, dass** die Polymerisation des Monomers in einem nach außen zur Atmosphäre gekapselten, hydraulisch gefüllten Behälter durchgeführt wird.

9. Verfahren nach Ansprüchen 1 bis 8, **dadurch gekennzeichnet, dass** der Mikrokapselaufbau aus einer Polymerprimärdispersion mit einer Dispergiereinheit erfolgt.

10. Verfahren nach Ansprüchen 1 bis 9, **dadurch gekennzeichnet, dass** der Mikrokapselaufbau aus einer Polymerprimärdispersion mit einem Rotor-Stator-System erfolgt.

11. Verfahren nach Ansprüchen 1 bis 10, **dadurch gekennzeichnet, dass** der Mikrokapselaufbau aus einer Polymerprimärdispersion mit einem Rotor-Stator-System in der Weise erfolgt, dass das über dem Reaktionsgemisch stehende Gas eingetragen wird (Selbstbegasung) und/oder dass aktiv Gas in das Reaktionsgemisch eingetragen wird (Fremdbegasung).

12. Verfahren nach Ansprüchen 1 bis 11, **dadurch gekennzeichnet, dass** der Mikrokapselaufbau aus einer Polymerprimärdispersion mit einem Rotor-Stator-System erfolgt, das in einem Rührkessel mit einem Verhältnis Durchmesser zu Höhe von 0,3 bis 2,5 angeordnet ist.

13. Verfahren nach Ansprüchen 1 bis 12, **dadurch gekennzeichnet, dass** der Mikrokapselaufbau aus einer Polymerprimärdispersion mit einem Rotor-Stator-System erfolgt, das in der Außenschlaufe eines Schlaufenreaktors angeordnet ist.

14. Verfahren nach Ansprüchen 1 bis 13, **dadurch gekennzeichnet, dass** eines oder mehrere der folgenden Monomere eingesetzt werden: Lactide, Alkylester der Acrylsäure, Alkylester der Methacrylsäure, und bevorzugt Alkylester der Cyanacrylsäure.

15. Verfahren nach Ansprüchen 1 bis 14, **dadurch gekennzeichnet, dass** eines oder mehrere der folgenden Monomere eingesetzt werden:
Butyl-, Ethyl- und Isopropylcyanacrylsäure.

16. Verfahren nach Ansprüchen 1 bis 15, **dadurch gekennzeichnet, dass** das oder die Monomere in einer Konzentration von 0,1 bis 60 %, bevorzugt von 0,1 bis 10 % zur sauren wäßrigen Lösung zugesetzt werden.

17. Verfahren nach Ansprüchen 1 bis 16, **dadurch gekennzeichnet, dass** ein oder mehrere der folgenden Tenside eingesetzt werden:
Alkylarylpoly(oxyethylen)sulfat Alkali-Salze, Dextrane, Poly(oxethylene), Poly(oxypropylen)-poly(oxyethylen)-Blockpolymere, ethoxylierte Fettalkohole (Cetomacrogole), ethoxylierte Fettsäuren, Alkylphenolpoly(oxyethylene), Copolymere von Alkylphenolpoly(oxyethylen)en und Aldehyden, Partialfettsäureester des Sorbitans, Partialfettsäureester des Poly(oxyethylen)sorbitans, Fettsäureester des Poly(oxyethylen)s, Fettalkoholether des Poly(oxyethylen)s, Fettsäureester der Saccharose oder Macrogolglycerolester, Polyvinylalkohole, Poly(oxyethylen)-hydroxyfettsäureester, Macrogole mehrwertiger Alkohole, Partialfettsäureester.

18. Verfahren nach Ansprüchen 1 bis 16, **dadurch gekennzeichnet, dass** ein oder mehrere der folgenden Tenside eingesetzt werden:
Ethoxylierte Nonylphenole, ethocylierte Octylphenole, Copolymer von Aldehyden und Octylphenolpoly(oxyethylen), ethoxylierte Glycerin-Partialfettsäureester, etoxyliertes hydriertes Rizinusöl, Poly(oxyethylen)-hydroxystearat, Poly(oxypropylen)-poly(oxyethylen)-Blockpolymere mit einer Molmasse <20000.

19. Verfahren nach Ansprüchen 1 bis 16, **dadurch gekennzeichnet, dass** ein oder mehrere der folgenden Tenside eingesetzt werden:
para-Octylphenol-poly-(oxyethylen) mit im Mittel 9-10 Ethoxygruppen (=Octoxynol 9,10), para-Nonylphenol-poly(oxyethylen) mit im Mittel 30/40 Ethoxygruppen (=z.B. Emulan®30,/Emulan®40), para-Nonylphenolpoly(oxyethylen)-sulfat-Na Salz mit im Mittel 28 Ethoxygruppen (= z.B. Disponil® AES), Poly(oxyethylen)glycerinmonostearat (z.B. Tagat® S), Polyvinylalkohol mit Polymerisationsgrad von 600-700 und einem Hydrolysegrad 85 % - 90 % (= z.B. Mowiol® 4-88), Pol(oxyyethylen)-660-Hydrocystearinsäureester (= z.B. Solutol® HS 15), Copolymer von Formaldehyd und para-Octylphenolpoly(oxyethylen) (= z.B. Triton® WR 1339), Polyoxypropylen-polyoxyethylen-Blockpolymere mit einer Molmasse von ca. 12000 und einem Polyoxyethylen-Anteil von ca. 70 % (= z.B. Lutrol® F127), ethoxylierter Cetylstearylalkohol (= z.B. Cremophor®) A25), ethoxyliertes Rizinusöl (= z.B. Cremophor® EL).

20. Verfahren nach Ansprüchen 1 bis 19, **dadurch gekennzeichnet, dass** das oder die Tenside in einer Konzentration von 0,1 bis 10 % eingesetzt werden.

21. Verfahren nach Ansprüchen 1 bis 20, **dadurch gekennzeichnet, dass** mindestens einer der Verfahrensschritte in saurer wässriger Lösung durchgeführt wird.

22. Verfahren nach Ansprüchen 1 bis 21, **dadurch gekennzeichnet, dass** folgende Säuren eingesetzt werden: Salzsäure, Phosphorsäure und/oder Schwefelsäure.

23. Verfahren nach Ansprüchen 1 bis 22, **dadurch gekennzeichnet, dass** der oder die Zusatzstoffe in einer Konzentration von 0,1 bis 10 % eingesetzt werden.

24. Verfahren nach Ansprüchen 1 bis 23, **dadurch gekennzeichnet, dass** die Polymerisation und der Mikrokapselaufban bei Temperaturen von -10 °C bis 60 °C, bevorzugt im Bereich zwischen 0 °C und 50 °C, besonders bevorzugt zwischen 10 °C und 35 °C, erfolgt.

25. Verfahren nach Ansprüchen 1 bis 24, **dadurch gekennzeichnet, dass** die gasgefüllten Mikrokapseln vom Reaktionsmedium abetrennt werden, in einem physiologisch verträglichen Medium aufgenommen und ggf. nach Zusatz eines Kryoprotektors gefriergetrocknet werden.

26. Verfahren nach Ansprüchen 1 bis 25, **dadurch gekennzeichnet, dass** zur Aufnahme des Flotats Wasser oder 0,9 % Kochsalzlösung als physiologisch verträgliches Medium verwendet wird.

27. Verfahren nach Ansprüchen 1 bis 26, **dadurch gekennzeichnet, dass** als Kryoprotektor Polyvinylpyrrolidon, Polyvinylalkohol, Gelatine und/oder Humanserumalbumin verwendet wird.

## Claims

1. Multistage process for the preparation of gas-filled microcapsules, **characterized in that** the following process steps are carried out spatially and/or temporally separate
a) polymerization of the enveloping substance with formation of a primary dispersion of colloidal polymer particles
b) structure-building aggregation of the colloidal polymer particles to give gas-filled microcapsules.

2. Process according to Claim 1, **characterized in that** the polymerization of the monomer is carried out in aqueous solution under stirring conditions such that the gas phase content in the stirring medium is < 1%.

3. Process according to Claim 1 or 2, **characterized in that** the microcapsule synthesis is carried out from a polymer primary dispersion under dispersing conditions such that the gas phase content in the dispersing medium is > 1%, preferably greater than 10%.

4. Process according to one of Claims 1 to 3, **characterized in that** the polymerization of the monomer is carried out in a batchwise, semi-continuous or continuous stirring vessel having a diameter to height ratio from 0.3 to 2.5.

5. Process according to one of Claims 1 to 4, **characterized in that** the polymerization of the monomer is carried out in a batchwise, semi-continuous or continuous stirring vessel in a diameter to height ratio from 0.3 to 2.5 having an external loop (loop reactor), in which is arranged a single- or multistage dispersing unit which is connected at the start of the reaction or later.

6. Process according to Claims 1 to 5, **characterized in that** the polymerization of the monomer is carried out in a stirring element which is arranged vertically, inclined or at the side, whose diameter in relation to the reactor diameter lies in a range from 0.2 to 0.7.

7. Process according to Claims 1 to 3, **characterized in that** the polymerization of the monomer is carried out in a continuously operated flow tube reactor.

8. Process according to Claims 1 to 7, **characterized in that** the polymerization of the monomer is carried out in a hydraulically filled container enclosed outwardly from the atmosphere.

9. Process according to Claims 1 to 8, **characterized in that** the microcapsule synthesis is carried out from a polymer primary dispersion using a dispersing unit.

10. Process according to Claims 1 to 9, **characterized in that** the microcapsule synthesis is carried out from a polymer primary dispersion using a rotor-stator system.

11. Process according to Claims 1 to 10, **characterized in that** the microcapsule synthesis is carried out from a polymer primary dispersion using a rotor-stator system in such a way that the gas above the reaction mixture is introduced (self-gasification) and/or **in that** active gas is introduced into the reaction mixture (independent gasification).

12. Process according to Claims 1 to 11, **characterized in that** the microcapsule synthesis is carried out from a polymer primary dispersion using a rotor-stator system which is arranged in a stirring vessel having a diameter to height ratio from 0.3 to 2.5.

13. Process according to Claims 1 to 12, **characterized in that** the microcapsule synthesis is carried out from a polymer primary dispersion using a rotor-stator system which is arranged in the external loop of a loop reactor.

14. Process according to Claims 1 to 13, **characterized in that** one or more of the following monomers are employed: lactides, alkyl esters of acrylic acid, alkyl esters of methacrylic acid, and preferably alkyl esters of cyanoacrylic acid.

15. Process according to Claims 1 to 14, **characterized in that** one or more of the following monomers are employed:
butyl-, ethyl- and isopropylcyanoacrylic acid.

16. Process according to Claims 1 to 15, **characterized in that** the monomer or the monomers is or are added to the acidic aqueous solution in a concentration from 0.1 to 60%, preferably from 0.1 to 10%.

17. Process according to Claims 1 to 16, **characterized in that** one or more of the following surfactants are employed:
alkylarylpoly(oxyethylene)sulphate alkali metal salts, dextrans, poly(oxyethylenes), poly(oxypropylene)-poly(oxyethylene) block polymers, ethoxylated fatty alcohols (cetomacrogols), ethoxylated fatty acids, alkylphenolpoly(oxyethylenes), copolymers of alkylphenolpoly(oxyethylene)s and aldehydes, partial fatty acid esters of sorbitan, partial fatty acid esters of poly(oxyethylene)sorbitan, fatty acid esters of poly(oxyethylene), fatty alcohol ethers of poly(oxyethylene), fatty acid esters of sucrose or macrogol glycerol esters, polyvinyl alcohols, poly(oxyethylene)hydroxy fatty acid esters, macrogols of polyhydric alcohols, partial fatty acid esters.

18. Process according to Claims 1 to 16, **characterized in that** one or more of the following surfactants are employed:
ethoxylated nonylphenols, ethoxylated octylphenols, copolymers of aldehydes and octylphenol-poly(oxyethylene), ethoxylated glycerol partial fatty acid esters, ethoxylated hydrogenated castor oil, poly(oxyethylene) hydroxystearate, poly(oxypropylene)-poly(oxyethylene) block polymers having a molar mass of < 20,000.

19. Process according to Claims 1 to 16, **characterized in that** one or more of the following surfactants are employed:
para-octylphenol-poly(oxyethylene) having on average 9-10 ethoxy groups (= octoxynol 9,10), para-nonylphenol-poly(oxyethylene) having on average 30/40 ethoxy groups (= e.g. Emulan®30/Emulan®40), para-nonylphenol-poly(oxyethylene) sulphate Na salt having on average 28 ethoxy groups (= e.g. Disponil® AES), poly(oxyethylene)-glycerol monostearate (e.g. Tagat® S), polyvinyl alcohol having a degree of polymerization of 600-700 and a degree of hydrolysis of 85%-90% (= e.g. Mowiol® 4-88), poly(oxyethylene)-660 hydroxystearic acid ester (= e.g. Solutol® HS 15), copolymer of formaldehyde and para-octylphenolpoly(oxyethylene) (= e.g. Triton® WR 1339), polyoxypropylene-polyoxyethylene block polymers having a molar mass of about 12,000 and a polyoxyethylene content of about 70% (= e.g. Lutrol® F127), ethoxylated cetylstearyl alcohol (= e.g. Cremophor® A25), ethoxylated castor oil (= e.g. Cremophor® EL).

20. Process according to Claims 1 to 19, **characterized in that** the surfactant or the surfactants is or are employed in a concentration from 0.1 to 10%.

21. Process according to Claims 1 to 20, **characterized in that** at least one of the process steps is carried out in acidic aqueous solution.

22. Process according to Claims 1 to 21, **characterized in that** the following acids are employed:
hydrochloric acid, phosphoric acid and/or sulphuric acid.

23. Process according to Claims 1 to 22, **characterized in that** the additive or the additives is or are employed in a concentration from 0.1 to 10%.

24. Process according to Claims 1 to 23, **characterized in that** the polymerization and the microcapsule synthesis are carried out at temperatures from -10°C to 60°C, preferably in the range between 0°C and 50°C, particularly preferably between 10°C and 35°C.

25. Process according to Claims 1 to 24, **characterized in that** the gas-filled microcapsules are separated from the reaction medium, taken up in a physiologically tolerable medium and, optionally after addition of a cryoprotector, are freeze-dried.

26. Process according to Claims 1 to 25, **characterized in that**, for taking up the flotate, water or 0.9% saline solution is used as the physiologically tolerable medium.

27. Process according to Claims 1 to 26, **characterized in that** the cryoprotector used is polyvinylpyrrolidone, polyvinyl alcohol, gelatin and/or human serum albumin.

## Revendications

1. Procédé en plusieurs étapes pour la préparation de microcapsules remplies de gaz, **caractérisé en ce qu'**on effectue, séparément dans l'espace et/ou dans le temps, les étapes suivantes de processus
a) polymérisation de la substance constituant l'enveloppe avec formation d'une dispersion primaire de particules colloïdales de polymère,
b) agrégation, constituant la structure, des particules colloïdales de polymère en microcapsules remplies de gaz.

2. Procédé selon la revendication 1, **caractérisé en ce que** la polymérisation du monomère est effectuée en solution aqueuse dans des conditions d'agitation telles que la proportion de phase gazeuse dans le milieu d'agitation est < 1 %.

3. Procédé selon la revendication 1 ou 2, **caractérisé en ce que** l'élaboration de microcapsules à partir d'une dispersion primaire de polymère s'effectue dans des conditions de dispersion telles que la proportion de phase gazeuse dans le milieu de dispersion est > 1 %, de préférence supérieure à 10 %.

4. Procédé selon l'une quelconque des revendications 1 à 3, **caractérisé en ce que** la polymérisation du monomère est effectuée dans un récipient à agitation en mode discontinu, semi-continu ou continu, ayant un rapport du diamètre à la hauteur de 0,3 à 2,5.

5. Procédé selon l'une quelconque des revendications 1 à 4, **caractérisé en ce que** la polymérisation du monomère est effectuée dans un récipient à agitation en mode discontinu, semi-continu ou continu, ayant un rapport du diamètre à la hauteur de 0,3 à 2,5, avec boucle externe (réacteur à boucle), dans lequel est disposée une unité de dispersion à un ou plusieurs étages, qui est mis en action au début de la réaction ou ultérieurement.

6. Procédé selon les revendications 1 à 5, **caractérisé en ce que** la polymérisation du monomère est effectuée dans un organe d'agitation disposé verticalement, obliquement ou latéralement, dont le diamètre par rapport au diamètre du réacteur est dans une plage allant de 0,2 à 0,7.

7. Procédé selon les revendications 1 à 3, **caractérisé en ce que** la polymérisation du monomère est effectuée dans un réacteur tubulaire à écoulement fonctionnant en continu.

8. Procédé selon les revendications 1 à 7, **caractérisé en ce que** la polymérisation du monomère est effectuée dans un récipient à remplissage hydraulique isolé de l'atmosphère vers l'extérieur.

9. Procédé selon les revendications 1 à 8, **caractérisé en ce que** l'élaboration des microcapsules à partir d'une dispersion primaire de polymère s'effectue à l'aide d'une unité de dispersion.

10. Procédé selon les revendications 1 à 9, **caractérisé en ce que** l'élaboration des microcapsules à partir d'une dispersion primaire de polymère s'effectue à l'aide d'un système rotor-stator.

11. Procédé selon les revendications 1 à 10, **caractérisé en ce que** l'élaboration des microcapsules à partir d'une dispersion primaire de polymère s'effectue à l'aide d'un système rotor-stator de telle façon que le gaz se trouvant au-dessus du mélange réactionnel est introduit (auto-introduction de gaz) et/ou que le gaz actif dans le mélange réactionnel est introduit (hétéro-introduction de gaz).

12. Procédé selon les revendications 1 à 11, **caractérisé en ce que** l'élaboration des microcapsules à partir d'une dispersion primaire de polymère s'effectue à l'aide d'un système rotor-stator qui est disposé dans un récipient à agitation ayant un rapport du diamètre à la hauteur de 0,3 à 2,5.

13. Procédé selon les revendications 1 à 12, **caractérisé en ce que** l'élaboration des microcapsules à partir d'une dispersion primaire de polymère s'effectue à l'aide d'un système rotor-stator qui est disposé dans la boucle externe d'un réacteur à boucle.

14. Procédé selon les revendications 1 à 13, **caractérisé en ce qu'**on utilise un ou plusieurs des monomères suivants : lactides, esters alkyliques de l'acide acrylique, esters alkyliques de l'acide méthacrylique et de préférence esters alkyliques de l'acide cyano-acrylique.

15. Procédé selon les revendications 1 à 14, **caractérisé en ce qu'**on utilise un ou plusieurs des monomères suivants :
acides butylcyanoacrylique, éthylcyanoacrylique et isopropylcyanoacrylique.

16. Procédé selon les revendications 1 à 15, **caractérisé en ce que** le ou les monomères sont ajoutés à la solution aqueuse acide à une concentration de 0,1 à 60 %, de préférence de 0,1 à 10 %.

17. Procédé selon les revendications 1 à 16, **caractérisé en ce qu'**on utilise un ou plusieurs des tensioactifs suivants :
alkylarylpoly(oxyéthylène)sulfates de métaux alcalins, dextranes, poly(oxyéthylène)s, polymères séquencés poly(polyoxypropylène)/poly(oxyéthylène), alcools gras éthoxylés (cétomacrogols), acides gras éthoxylés, alkylphénolpoly(oxyéthylène)s, copolymères d'alkylphénolpoly(oxyéthylène)s et d'aldéhydes, esters partiels d'acides gras du sorbitanne, esters partiels d'acides gras du poly(oxyéthylène)sorbitanne, esters d'acides gras du poly(oxyéthylène), éthers d'alcools gras du poly(oxyéthylène), esters d'acides gras du saccharose ou esters de macrogols et de glycérols, poly(alcool vinylique)s, esters polyoxyéthyléniques d'acides gras hydroxylés, macrogols d'alcools polyhydroxylés, esters partiels d'acides gras.

18. Procédé selon les revendications 1 à 16, **caractérisé en ce qu'**on utilise un ou plusieurs des tensioactifs suivants :
nonylphénols éthoxylés, octylphénols éthoxylés, copolymère d'aldéhydes et d'octylphénolpoly(oxyéthylène), esters partiels d'acides gras et de glycérol éthoxylés, huile de ricin éthoxylée hydrogénée, poly(oxyéthylène)-hydroxystéarate, polymères séquencés poly(oxypropylène)/poly(oxyéthylène) ayant une masse moléculaire < 20 000.

19. Procédé selon les revendications 1 à à 16, **caractérisé en ce qu'**on utilise un ou plusieurs des tensioactifs suivants :
para-octylphénylpoly(oxyéthylène) ayant en moyenne 9-10 groupes éthoxy (= octoxynol-9,10), paranonylphényl-poly(oxyéthylène) ayant en moyenne 30/40 groupes éthoxy (= par exemple Emulan® 30/Emulan® 40), paranonylphénol-poly(oxyéthylène)-sulfate de Na ayant en moyenne 28 groupes éthoxy (= par exemple Disponil® AES), mono-stéarate polyoxyéthylénique de glycérol (= par exemple Tagat® S), poly(alcool vinylique) ayant un degré de polymérisation de 600-700 et un degré d'hydrolyse de 85 %-90 % (= par exemple Mowiol® 4-88), poly(oxyéthylène)-660-ester d'acide hydroxystéarique (= par exemple Solutol® HS 15), copolymère de formaldéhyde et para-octylphénolpoly(oxyéthylène) (= par exemple Triton® WR 1339), polymères séquencés polyoxypropylène/polyoxyéthylène ayant une masse moléculaire d'environ 12 000 et une teneur en polyoxyéthylène d'environ 70 % (= par exemple Lutrol® F127), alcool cétylstéarylique éthoxylé (= par exemple Cremophor® A25), huile de ricin éthoxylée (= par exemple Cremophor® EL).

20. Procédé selon les revendications 1 à à 19, **caractérisé en ce que** le ou les tensioactifs sont utilisés à une concentration de 0,1 à 10 %.

21. Procédé selon les revendications 1 à à 20, **caractérisé en ce qu'**au moins l'une des étapes du processus est effectuée en solution aqueuse acide.

22. Procédé selon les revendications 1 à 21, **caractérisé en ce qu'**on utilise les acides suivants : acide chlorhydrique, acide phosphorique et/ou acide sulfurique.

23. Procédé selon les revendications 1 à 22, **caractérisé en ce que** le ou les additifs sont utilisés à une concentration de 0,1 à 10 %.

24. Procédé selon les revendications 1 à 23, **caractérisé en ce que** la polymérisation et l'élaboration des microcapsules sont effectuées à des températures de -10°C à +60°C, de préférence dans la plage comprise entre 0°C et 50°C, de façon particulièrement préférée entre 10°C et 35°C.

25. Procédé selon les revendications 1 à à 24, **caractérisé en ce que** les microcapsules remplies de gaz sont séparées du milieu réactionnel, reprises dans un milieu physiologiquement acceptable et éventuellement, après addition d'un cryoprotecteur, lyophilisées.

26. Procédé selon les revendications 1 à 25, **caractérisé en ce que** pour la reprise du produit flottant, on utilise comme milieu physiologiquement acceptable de l'eau ou une solution de chlorure de sodium à 0,9 %.

27. Procédé selon les revendications 1 à 26, **caractérisé en ce qu'**on utilise comme cryoprotecteur la polyvinylpyrrolidone, le poly(alcool vinylique), la gélatine et/ou l'albumine de sérum humain.
